# EUROPEAN PATENT APPLICATION

(11) **EP 3 906 882 A1**
(43) Date of publication of application: **10.11.2021**
(21) Application number: 20305445.7
(22) Date of filing: 05.05.2020
(51) Int. Cl.: A61B 50/13, A61B 34/20, A61B 17/16, A61B 34/30, A61B 90/50

(54) **SURGICAL ROBOTIC SYSTEM**

(71) Applicant: Ecential Robotics, 38610 Gieres (FR)
(72) Inventor: LAVALLEE, Stéphane, 38410 SAINT-MARTIN-D'URIAGE (FR); LEON, Adrien, 38610 GIERES (FR); BELLY, Christian, 38610 GIERES (FR); FOURNIER, Elie, 38360 SASSENAGE (FR)
(74) Representative: Regimbeau

(57) **Abstract**

The invention relates to a surgical robotic system (1) to reach a plurality of surgical targets inside a target space, said system comprising:
- a robotic arm (4) having at least six degrees of freedom, the robotic arm comprising a base (40) located at a proximal end and a plurality of consecutive segments interconnected by a plurality of joints extending from the base (40) to a distal end, and said robotic arm having a first joint center (O) between the base (40) and the first segment (41),
- an end effector (5) for holding a medical device such as a surgical instrument or a surgical tool guide, said end effector being mechanically coupled to the robotic arm distal end,
- a movable cart (2) having a frontal side (20) comprising at least one frontal surface configured to be placed against a side (60) of an operating table (6), defining a horizontal axis (Ax) with a direction (x) orthogonal to the frontal surface and an origin point A located on the frontal surface such that all points located on the movable cart side have a negative coordinate on said axis (Ax), and all points located on the operating table side have a positive coordinate on said axis (Ax),
- a rigid elongated neck (3) comprising a proximal end mechanically coupled to the movable cart and a distal end mechanically coupled to the first segment (41) of the robotic arm,
- a control unit (7) configured to controllably move the robotic arm,
characterized in that the neck (3) is configured such that the coordinate (K) of the first joint center (O) on the axis (Ax) is strictly positive.

## Description

### FIELD OF THE INVENTION

The present invention relates to the field of robotic surgery. In particular, the present invention relates to the field of systems with a movable cart for a robotic arm to be used during a surgical operation.

Typical surgical operations using robotic surgery include: implantation of orthopedic implants such as pedicular screws in the spine, implantation of various orthopedic implants in bones, reduction and fixation of fractures during traumatological procedures, or just positioning guides or canulae at a desired position with respect to a predetermined target.

More particularly, the present invention can be used for maintaining targets for pedicle screw placement defined in a preoperative or intraoperative image-based planning step.

### BACKGROUND OF THE INVENTION

Surgical robotic systems are already used in assisting a user (e.g. a surgeon) during a surgical intervention.

For example, in spine surgery, the user may have to implant one or several screws into several vertebrae. A robotic arm may assist the user by holding a drill guide and by maintaining said drill guide according to a planned axis. The user thus uses a handheld drill passing through the drill guide held by the robotic arm to drill a hole intended to receive the screw in a vertebra along the planned axis.

Usually, the robotic systems according to the prior art comprise a robotic arm having a proximal end extending from a base, which usually is a movable cart (ROSA™ from ZIMMER BIOMET, EXCELCIUS GPS™ from GLOBUS MEDICAL). Said movable cart is brought near the operating table, and the robotic arm is then deployed according to the needs of the surgeon.

Limitations of such solutions arise from the use and usability of such surgery robotic arms. Indeed, due to their limited range and limited joint freedoms, points of interest are not always reachable. Another drawback is that known robotic arms may have a big size to reach all target positions, which requires large motors on each axis, and high weight of robot segments, thus creating a high degree of inertia of such robotic devices, said inertia being a limiting factor to high speed servo control of the robotic arm, thus limiting the use and indications of such robotic arms.

Depending on numerous conditions such as: the length of the robot i.e. the cumulative segment lengths, the capabilities i.e. range of motion at each joint for example, the surgical conditions (width of the operating table, position of the patient, size and weight of the patient, type and goal of the surgery), situations could happen where the robotic arm cannot properly reach a target, either automatically when this target has been determined during planning or manually by a manual guiding of the surgeon or a dedicated operator.

A straightforward solution could be to use a robotic arm having greater dimensions, i.e. greater segments, greater ranges of motion at the joints and/or more degrees of freedom. But such solution comes with a severe drawback that is the negative impact on the robotic arm overall accuracy during real-time servo control. Such accuracy is of utmost importance in surgical operations with surgical robotic systems for obvious safety reasons. Furthermore, the robotic arm stiffness, i.e. the capacity of the robotic arm to resist kinematic deformation in response to an applied force, a critical parameter regarding the use of robotic arms in a surgical procedure, is also negatively impacted by such a solution.

Another solution is to attach the robot base to the operating table such as in MAZOR X™ robotic device (MEDTRONIC). But attaching the robotic system to the table introduces new stability issues since the operating table rails may not be stiff enough, and a lack of flexibility for removing or using the robot at will.

### BRIEF DESCRIPTION OF THE INVENTION

It is an object of this invention to provide a robotic arm system that allows using a smaller, lighter robotic arm for surgical operation needs, while allowing a wider range of surgical targets to be reached.

To that end, the invention provides a robotic surgical robotic system to reach a plurality of surgical targets inside a target space, said system comprising:
- a robotic arm having at least six degrees of freedom, the robotic arm comprising a base located at a proximal end and a plurality of consecutive segments interconnected by a plurality of joints extending from the base to a distal end, and said robotic arm having a first joint center between the base and the first segment,
- an end effector for holding a medical device such as a surgical instrument or a surgical tool guide, said end effector being mechanically coupled to the robotic arm distal end,
- a movable cart having a frontal side comprising at least one frontal surface configured to be placed against a side of an operating table, defining a horizontal axis Ax with a direction x orthogonal to the frontal surface and an origin point A located on the frontal surface such that all points located on the movable cart side have a negative coordinate on said Ax axis, and all points located on the operating table side have a positive coordinate on said Ax axis,
- a rigid elongated neck comprising a proximal end mechanically coupled to the movable cart and a distal end mechanically coupled to the first segment of the robotic arm,
- a control unit configured to controllably move the robotic arm,
characterized in that the neck is configured such that the coordinate of the first joint center on the axis is strictly positive.

In some preferred embodiments, the neck is configured such that the coordinate of the first joint center on the Ax axis is greater than +100 mm.

In some preferred embodiments, the neck is configured such that a coordinate of a shoulder point, which is defined as the intersection point between the first and second axes of the robotic arm, along the axis is comprised between minimum and maximum coordinates of the target space on the axis.

In some embodiments, the mechanical coupling between the neck and the movable cart is configured to enable a movement of the neck relative to the movable cart according to at least one degree of freedom.

Said mechanical coupling between the neck and the movable cart may be configured to enable a translation of the neck along a vertical axis and a rotation of the neck about said vertical axis (z) relative to the movable cart.

In some embodiments, the mechanical coupling between the neck and the movable cart comprises at least one motor configured to drive a movement of the neck relative to the movable cart according to said at least one degree of freedom.

In some embodiments, the mechanical coupling between the neck and the movable cart is configured to enable a motorized vertical translation of the neck and a manual rotation of the neck around the vertical axis.

Advantageously, the vertical axis of the neck is used to rotate by 180 degrees the neck and robotic arm such that the neck and robotic arm are inside the footprint of the mobile cart for transportation.

In some embodiments, the control unit may also be configured to controllably move the motorized motions of the cart-neck mechanical coupling.

In some embodiments, the mechanical coupling between the neck and the movable cart comprises at least one abutment and at least one locking means cooperating with the abutment to stop and lock the neck relative to the movable cart in a determined configuration.

In some embodiments, the control unit may further be configured to detect at least one safety configuration of the movable cart and/or robotic system.

Said at least one safety configuration may comprise the position of the neck and robotic arm within a footprint of the movable cart.

In some embodiment, the system may further comprise a safety fence movable between an unfolded protective position wherein the safety fence surrounds the robotic arm and a folded position wherein the safety fence enables operation of the robotic arm, wherein the at least one safety parameter comprises the unfolded position of safety fence.

Preferably, the control unit may allow displacement of the movable cart on a distance greater than two meters only when said control unit detects that said at least one safety configuration is activated.

In some embodiments, the neck comprises a curved portion between its proximal end and its distal end.

In other embodiments, the neck may present a straight shape between its proximal end and its distal end with an inclination of 45 degrees with respect to the vertical.

In some embodiments, the neck distal end has an axis aligned with the first axis of the robotic arm.

### Definitions

In the present invention, the following terms have the following meanings:
- "A plurality" in the context of the invention means at least two.
- "Proximal end" in the context of the invention is used with reference to the movable cart, the proximal end being the end closest to the movable cart.
- "Distal end" in the context of the invention is used with reference to the movable cart, the distal end being the end farthest from the movable cart.
- "Approximately" in the context of the invention refers to a measure made considering an error margin that depends on the tool and measurement method used to obtain the value measured.
- "Horizontal plane" in the context of the invention refers to a plane strictly parallel to the ground of the operating room.
- "Vertical" in the context of the invention refers to a direction orthogonal to a horizontal plane.

### BRIEF DESCRIPTION OF THE DRAWINGS

Further features and advantages of the invention will be apparent from the following description, based on the appended drawings wherein:
- FIG. 1 represents a surgical scene including a robotic system according to an embodiment of the invention;
- FIG. 2 is a side view of the surgical scene of FIG. 1;
- FIG. 3 is a top view of the surgical scene of FIG. 1;
- FIG. 4 is a perspective view of the surgical system with the neck pivoted in a protective configuration;
- FIGS. 5A and 5B illustrate a first embodiment of a safety fence, respectively in an extended and a retracted configuration;
- FIGS. 6A and 6B illustrate a second embodiment of a safety fence, respectively in an extended and a retracted configuration;
- FIGS. 7A-7E illustrate various embodiments of the neck.

Reference signs that are identical from one figure to another one designates the same element, or elements fulfilling the same function.

### DETAILED DESCRIPTION OF EMBODIMENTS OF THE INVENTION

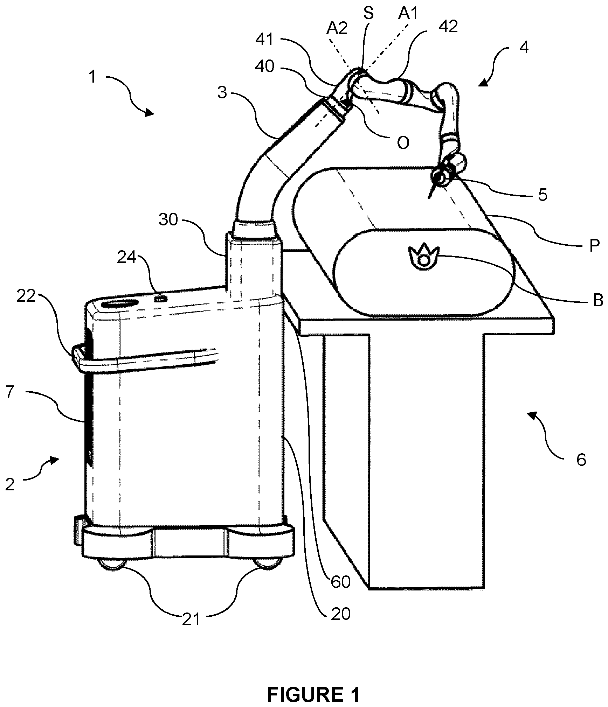

The invention is used in the context of robotic surgery such as depicted in the surgical scene of FIG. 1.

### Surgical robotic system

The surgical robotic system 1 has a movable cart 2 and a robotic arm 4 carried by the movable cart.

During a surgical operation, the cart is intended to remain fixed relative to an operating table on which a patient P lies, while the robotic arm is moved to reach the surgical targets. In particular, a frontal side 20 of the movable cart may be arranged to come as close as possible to the operating table, or up to be in contact with a side 60 of the operating table. The frontal side 20 extends substantially vertically. The frontal side may have a planar surface or may comprise at least one curved surface.

The movable cart may be mobile on wheels 21 and include at least one handle 22 to allow an operator to easily maneuver and transport the surgical robotic system 1. The handle 22 is advantageously located opposite the frontal side 20, so that the user may simply push the movable cart via the handle until being as close as possible to the operating table, up to contacting the side of the operating table.

The movable cart 2 may be manually actuated or, alternatively, motorized with at least one degree of freedom. At least one of the wheels may be blocked once the movable cart has been moved to the desired position against the operating table.

The robotic arm comprises a plurality of degrees of freedom in translation and/or rotation. Usually, the robotic arm comprises at least six motorized degrees of freedom. To that end, the robotic arm comprises a plurality of articulated segments driven by motors with encoders. By convention, the segments are numbered from the proximal end (which is the end closest to the movable cart) to the distal end of the robotic arm. The successive robotic arm joints may be rotations or translations. Successive rotations may be orthogonal or parallel. Some parts of the robotic arm may also use parallel mechanisms such as a hexapod architecture.

In a preferred embodiment, the robotic arm is made of six or seven rotation axis arranged in an anthropomorphic architecture, such as for example the KUKA liwa™ or LBR Med™, STAUBLI Puma 200™, or KINOVA Gen 3™. In such anthropomorphic architectures, the first and second axes are substantially orthogonal to each other.

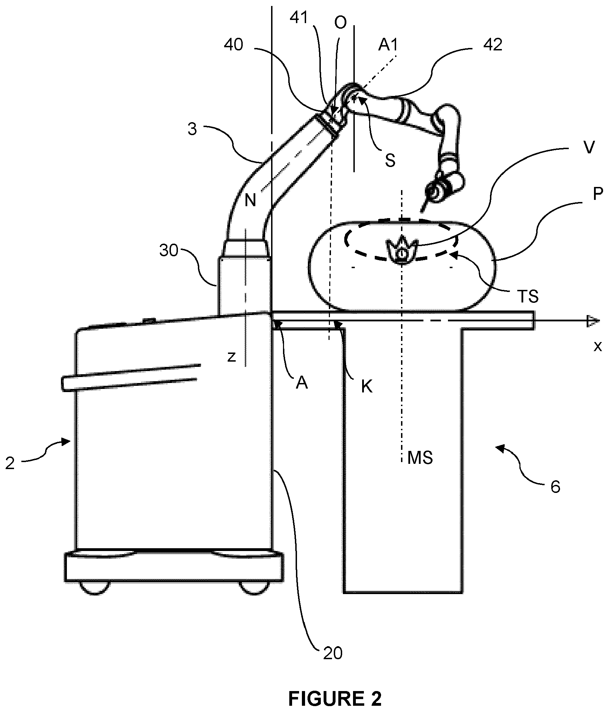

In FIGS. 1, 2 and 3, the robotic arm comprises a base 40, a first segment 41 and a second segment 42. The first segment 41 is pivotable relative to the base 40 about a first axis A1 and the second segment 42 is pivotable relative to the first segment about a second axis A2. In FIG. 2, the first axis A1 is in the plane of the figure and the second axis A2 is orthogonal to the plane of the figure.

Two characteristic points of the robotic arm may be defined as follows:
- the first joint center of the robotic arm, designated as point O, is a point of the first axis located at the center of the first joint, which is the joint between the base 40 and the first segment 41;
- the shoulder point of the robotic arm, designated as point S, is the intersection between the first axis 41 and the second axis 42, said intersection being defined as the closest point between said axes.

The robotic arm can be for example the LBR Med™ robot provided by KUKA (Germany). Said robotic arm may be either controlled in an autonomous mode according to desired targets and trajectories, or manipulated using a collaborative mode (cobot), or telemanipulated using a master control device. A combination of two or more of these different modes may be used on the same robotic system.

The robotic arm is advantageously designed to be as compact as possible, in order to increase the intrinsic mechanical stiffness of the robotic arm and reduce its inertia, so as to improve the behavior of the robotic arm when used with high speed servo control thanks to said reduced inertia.

The robotic arm 4 is coupled to the movable cart by an elongated rigid neck 3.

By "rigid" is meant in the present text that the neck may comprise several parts but that said parts are rigidly fixed relative to each other.

By "elongated" is meant in the present text that the neck has a length (measured along the neutral axis of the neck between the proximal end and the distal end of the neck) which is at least twice greater than the diameter of the neck. The neck may have a circular cross section; in this case, the diameter of the neck is the diameter of the cross section. If the neck does not have a circular cross section and/or if the neck does not have a constant cross section along its length, the "diameter" means in the present text the diameter of a disk having the same area as an average cross section of the neck.

The neck 3 comprises a proximal end coupled to the movable cart 2 by a mechanical coupling 30 and a distal end mechanically coupled to the proximal end (i.e. the base 40) of the robotic arm 4.

The robotic system comprises an end effector 5 for holding a medical device such as a surgical instrument or a surgical tool guide, said end effector being mechanically coupled to the distal end of the robotic arm 4.

The surgical robotic system 1 includes a control unit 7 configured to controllably move the robotic arm 4. In some embodiments, the control unit is configured to also controllably move at least part of the movable cart (e.g. at least one wheel). Advantageously, the control unit is also configured to controllably move the neck relative to the movable cart if such movements are possible and driven by motors.

The control unit 7 comprises a processor, a data storage device and a communication device. The control unit may advantageously be embedded in the movable cart 2. The movable cart may also comprise switches, such as power switches, an emergency button or the like. In other embodiments, the control unit may be provided separate from the movable cart, and may be configured to communicate wirelessly or by wires with the robotic arm.

Although not illustrated, the surgical scene may also include an X-ray imaging system, such as a C-arm for example, configured to acquire X-ray images of the patient before and/or during surgery.

The surgical scene may also include a localization system (not illustrated) configured to localize trackers attached to the patient, the robotic arm, the end-effector and/or the surgical tool or guide. The localization system may be chosen among various technologies, such as optical localization, electromagnetic localization, etc.

### Neck

The outer shape and size of the neck may be chosen to comply with stiffness and/or bulkiness requirements, taking into account that the neck supports the weight of the robotic arm. However, as mentioned above, the robotic arm is chosen to be as compact as possible and is thus relatively lightweight.

According to a preferred embodiment, the neck has a curved shape, or comprises at least one curved portion and at least one linear portion. In these cases, the neutral axis of the neck is at least partially curved. The distal end of the neck may have a greater inclination with respect to the vertical direction than the proximal end.

Alternatively, the neck may present a straight shape between its proximal end and its distal end. The neck axis may have an inclination of 45 degrees ± 5 degrees with respect to the vertical direction.

In other embodiments, the neck may comprise at least two linear portions inclined relative to each other.

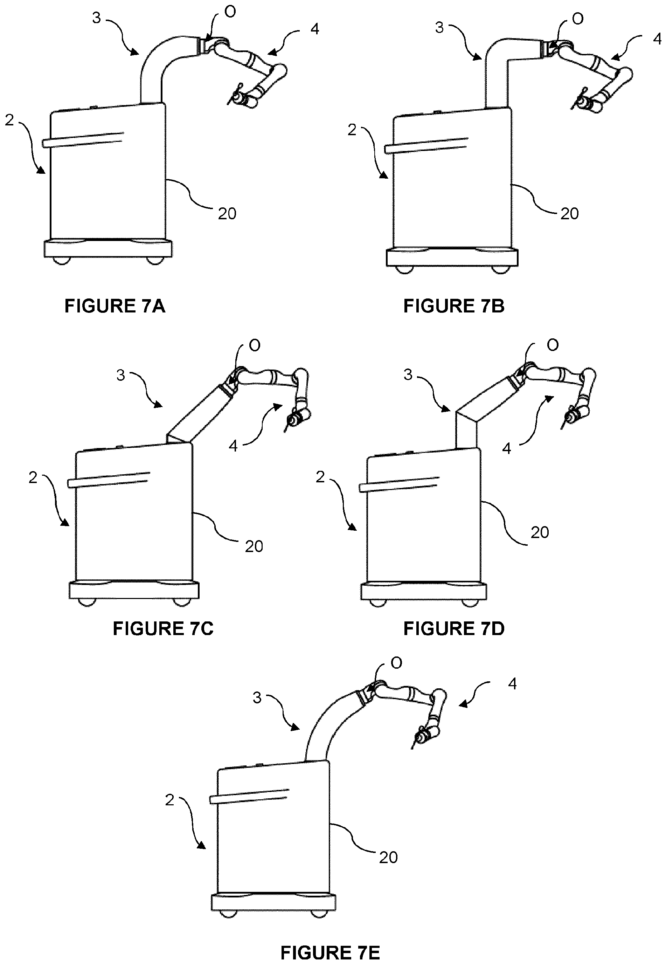

FIGS. 7A-7E illustrate some embodiments of the neck, which are not limitative. Each embodiment has specific advantages in terms of design, appearance, and is more or less cumbersome.

In the embodiment of FIGS. 7A and 7B, the neck 3 comprises a curved portion between linear proximal and distal portions, such that proximal and distal ends of the neck extend along substantially orthogonal axes (the proximal end extending along a vertical axis and the distal end extending along a horizontal axis). The radius of curvature of the curved portion is greater in the neck of FIG. 7A than in the neck of FIG. 7B.

In the embodiment of FIG. 7C, the neck 3 presents a straight shape.

In the embodiment of FIG. 7D, the neck 3 comprises two linear portions inclined with respect to each other, the proximal portion extending substantially along a vertical axis and the distal portion extending substantially along an axis inclined at about 45° relative to the vertical direction.

In the embodiment of FIG. 7E, the neck 3 has a curved shape with a greater radius of curvature than in FIGS. 7A and 7B, and the distal end does not extend along a horizontal axis but along an axis inclined at about 45° relative to the vertical direction.

The distal end of the neck 3 is rigidly fixed to the base 40 of the robotic arm. Advantageously, the axis N of the distal end of the neck is aligned with the axis of the base 40 of the robotic arm, which is usually colinear with the axis A1 of rotation of the first segment 41 relative to the base 40. Such an alignment provides a smooth continuity of the neck 3 and the robotic arm base 40. Said continuity is important for the perception of the user since it shows that it is a comprehensive and single system made of a non-robotic part for creating an extension towards the target space and a robotic part for creating a light weight servo-controlled system to adjust a surgical tool position. If the neck has a curved shape, said axis N of the distal end may be considered as being the axis of the mechanical coupling between the distal end of the neck and the base of the robotic arm.

In some embodiments, the proximal end of the neck 3 is rigidly fixed to the movable cart 2.

The mechanical coupling between the neck and the movable cart may be placed either on the top surface or the frontal surface of the movable cart. Alternatively, said mechanical coupling may be positioned on the edge between said top and frontal surfaces of the movable cart.

In other embodiments, the mechanical coupling between the proximal end of the neck and the movable cart is configured to enable at least one translation and/or at least one rotation of the neck relative to the movable cart. Said relative movement of the neck relative to the movable cart may in particular allow providing a transportation position of the robotic arm, wherein the robotic arm and the neck are retracted so as to substantially lie within the footprint of the movable cart, in particular beyond the frontal side 20 of the movable cart, and an operative position of the robotic arm, wherein the point O of the robotic arm is located outwardly from the frontal side 20. Said transportation position is particularly advantageous in that it avoids or at least limits the risk of hitting an obstacle with the robotic arm during a long displacement of the movable cart in the corridors and when passing doors. Such displacement being usually more than two meters.

In some embodiments, the neck may be movable manually by a user relative to the movable cart. The mechanical coupling between the neck and the movable cart may enable freely moving the neck within a determined range of motion. A button is used to lock the coupling in a given very rigid position. Mechanical, electrical, hydraulic, piezo electric or pneumatic brakes can be used for locking the vertical translation and/or rotation. Alternatively, the mechanical coupling may only enable a discrete number of predefined stable positions of the neck relative to the movable cart.

The rotation around the vertical axis of the proximal end of the neck can be used to adjust the position of the base 40 of the robotic arm along the operating table, towards the head or the feet of the patient. For example, a rotation of thirty degrees can shift the working space of the robotic arm without moving the mobile cart base in order to reach all desired targets of the target space.

In other embodiments, the mechanical coupling between the neck and the movable cart includes at least one motor configured to drive the neck in translation and/or in rotation relative to the movable cart. Said motor may be activated directly by the user, for example thanks to an actuation button provided on the movable cart, or by the control unit, for example if the control unit is configured to move the neck in specific situations.

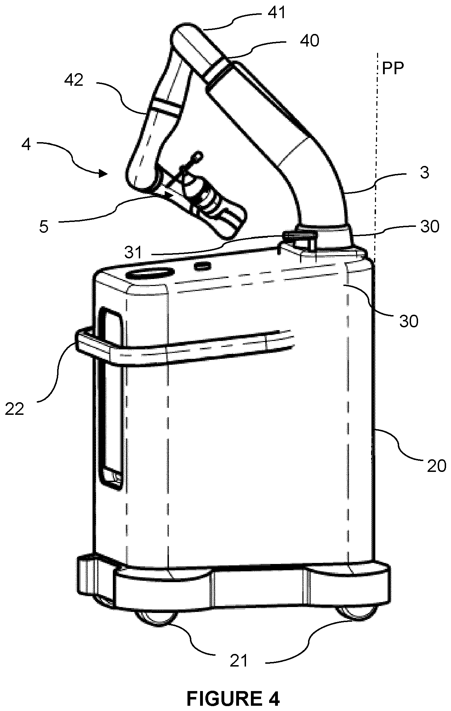

According to an embodiment, the mechanical coupling 30 between said neck and said movable cart comprises at least one abutment and at least one locking means 31 (see FIG. 4) cooperating with the abutment to stop and lock the neck relative to the movable cart in a desired configuration.

According to a preferred embodiment, the neck may be movable both in translation along a vertical axis and in rotation about said vertical axis z (see FIG. 2). The translation along the vertical axis may be driven by a motor (not shown) operable by a button 24 to be activated the user and/or by the control unit. The rotation about the vertical axis may be done manually. The movable cart may comprise at least one button (not shown) to be activated by the user to lock or unlock the rotation of the neck about the vertical axis. Advantageously, this configuration allows for maximal flexibility in the use of the surgical robotic system while keeping a relatively simple mechanical coupling between the neck and the movable cart.

As shown in FIGS. 1, 2, 3 and 7A-7E, the neck 3 extends laterally outwardly from the frontal surface 20 of the movable cart, so as to bring the first joint center O and the shoulder point S of the robotic arm at a non-null distance from said frontal surface of the movable cart.

### Target space

Usually, during the surgical intervention, not a single surgical target but a plurality of surgical targets has to be reached by the medical device held by the end effector to carry out the intended treatment (milling, drilling, sawing, placement of an implant, etc.). Said surgical targets may in particular consist in axes or planes along which the medical device is guided.

The plurality of surgical targets defines a target space. The target space may thus be considered as a volume defined relative to the patient's anatomy, the dimensions of said volume depending on the intended treatment.

For example, in spine surgery, the surgical targets may be defined relative to a plurality of vertebrae. For a complex surgery such as scoliosis of the spine, it may be necessary to operate more than ten vertebrae, which generate a very long target space along the patient's body. The target space may thus extend along the patient's spine, and may thus substantially extend along the mid-sagittal plane of the patient. During the surgical intervention, the mid-sagittal plane of the patient extends substantially along the longitudinal axis of the operating table. Said target space TS has been represented schematically on FIG. 2 around a vertebra V, centered on the mid-sagittal plane MS.

The working space of the robotic arm is a volume corresponding to all the positions reachable by the end effector attached at the distal end of the robotic arm. The working space depends on the dimension of the segments of the robotic arm and the range of motion of each joint of the robotic arm.

Moving the movable cart during the surgical intervention is not desired since it substantially increasing the operative time and creates risks of mistakes about sterility. The robotic arm and the cart are usually draped for preserving sterility of the surgical field. In order to reach all the targets without moving the movable cart, the working space of the robotic arm should enclose the target space.

The movable cart may thus be placed in a suitable position relative to the target space (which is at least approximately known by the user) to promote a favorable positioning of the working space relative to the target space.

The greater the dimensions of the robotic arm, the greater the working space and the easier the positioning of the robotic arm to reach the whole target space.

However, increasing the dimensions of the robotic arm segments may result in a reduced stiffness of the robotic arm and thus in a reduced accuracy of the positioning of the surgical targets, as well as an increased inertia which prevents highly accurate and fast servo control to track a moving surgical target, or perform a fast active motion of the robot end-effector to mill a bone on which a tracker has been attached for dynamic tracking and servo control at more than fifty Hertz.

On the other hand, a compact robotic arm, which presents a greater stiffness, also presents a reduced working space. In particular, the whole target space may not be reached if the first joint center O of the robotic arm is located too far from the target space.

The reduced working space provided by the compact robotic arm thus requires a closer positioning of the working space relative to the target space.

More precisely, said closer positioning may be obtained by placing the shoulder of the robotic arm closer to the center of the target space.

Since the movable cart is placed against a side of the operating table during the surgical intervention, the movable cart itself cannot be brought closer to the target space.

However, according to the invention, the elongated neck allows bringing the shoulder of the robotic arm closer to the target space, i.e. above the region of interest of the patient.

Thanks to the elongated neck, it is possible to impose a non-null distance between the shoulder and the frontal side of the movable cart.

### Positioning of the shoulder

When the frontal side 20 of the movable cart is placed against a side of the operating table (which may be a longitudinal side or a lateral side, depending on the anatomical region to be treated), a frontal surface is defined between the movable cart and the operating table. The frontal surface extends mainly in a vertical direction. Depending on the shape of the frontal side and its position and orientation relative to the operating table, the frontal surface may be a line, in particular a vertical line, or a plane, in particular a vertical plane.

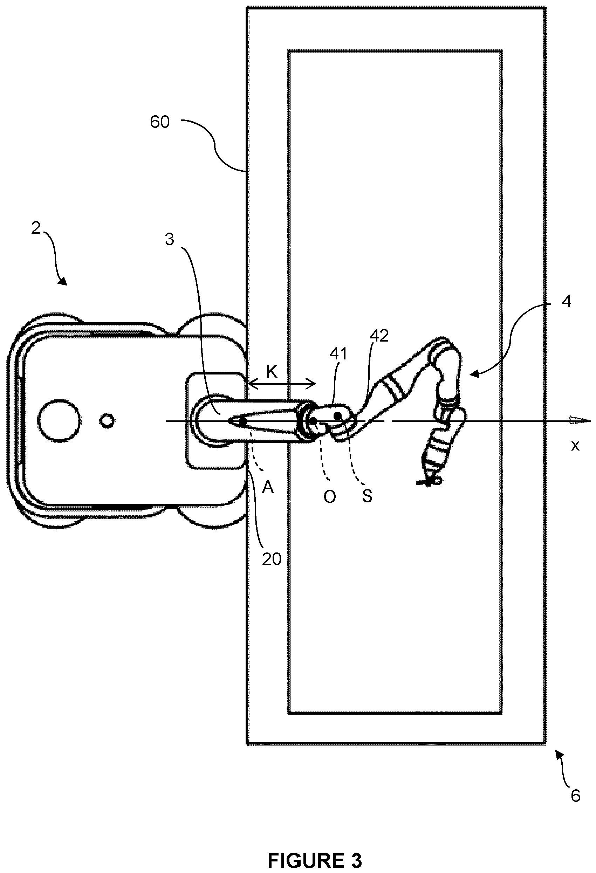

In both cases, as shown in FIGS. 2 and 3, it is possible to define an axis Ax extending in a horizontal direction x orthogonal to the frontal surface, i.e. orthogonal to a tangent to the frontal surface with an origin point A belonging to the frontal surface. The Ax axis is attached to the mobile cart; it is usually orthogonal to the side of the operating table when the robotic system is positioned against the operating table. However, the mobile cart can also be placed to make an angle with the side of the table, depending on user's preferences or operating room constraints. If the frontal surface is circular, such an angle will have no impact. If the frontal surface is flat, it will create an additional gap between the first joint center O of the robotic arm and the midline of the operating table, which may reduce the matching between the working space of the robot and the target space for some demanding applications, with a very compact robot and limited workspace.

A coordinate of a point on the Ax axis is determined by projecting said point orthogonally on the Ax axis.

The Ax axis is oriented such that all points located on the movable cart side have negative coordinate on said Ax axis, and all points located on the operating table side have a positive coordinate on said Ax axis.

Thanks to the elongated neck 3, the first joint center O has a strictly positive coordinate along the Ax axis. This has the advantage that all successive joints of the robotic arm are located above the operating table and therefore the working space of the robotic arm is closer to the target space, which reduces the requirements for a large and heavy robotic arm.

Preferably, the coordinate K of the first joint center O along the Ax axis is greater than or equal to +100 mm. Such value enables to offset significantly the kinematics of the robotic arm towards the target space in the case that the target space is centered on the operating table, which is the worst case.

In another embodiment, the shoulder point S has a coordinate which is comprised between the minimum and maximum coordinates of the target space on the Ax axis. Thanks to this location of the shoulder point, all targets of the target space may be reached easily by a compact robotic arm without moving the movable cart. This represents the optimal configuration wherein the shoulder point S representing the functional base of the robotic arm is centered above the target space. This offers an optimal match of the working space with the target space, thus enabling a compact design of the robotic arm, which has benefits in terms of accuracy, stiffness and real-time control.

According to a preferred embodiment, K is such that the shoulder point is approximately on the intersection between the mid-sagittal plane of the patient and the horizontal plane. This configuration is particularly interesting for spine surgery, since the surgical targets are defined relatively to the spine of the patient, which substantially extends along said mid-sagittal plane of the patient. The fact that the shoulder of the robotic arm is positioned above this region is particularly interesting for an optimal reach of all surgical targets near the spine of the patient while using a smaller robotic arm.

According to another embodiment, the shoulder point is still positioned approximately on the intersection between the mid-sagittal plane of the patient and the horizontal plane, but with the shoulder point positioned above the head of the patient, so as to easily reach surgical targets defined on cervical and upper thoracic vertebrae with high angles of orientations of the surgical tools.

According to an alternative embodiment, the shoulder point is still positioned approximately on the intersection between the mid-sagittal plane of the patient and the horizontal plane, but with the shoulder positioned above the sacral region of the patient, so as to easily reach surgical targets defined on lumbar and lower thoracic vertebrae.

Of course, the user may decide not to place the frontal side of the movable cart in contact with the operating table, but may keep a small gap (e.g. a few centimeters) between the frontal surface of the movable cart and the side of the operating table. In such case, the position of the shoulder point may be less favorable than in case of a contact between the movable cart and the operating table, since said gap generates an offset between the optimal position of the shoulder point provided by the neck and its real position relative to the target space. Provided that said gap remains less than the distance K, the neck according to the invention is still advantageous in that it brings the shoulder point closer to the target space.

The compact robot designed according the method described above is optimized for difficult geometries to reach, in particular in the center of the operating table. But for more simple cases, the mobile cart can be also placed at any position, at several tenths of centimeters from the operating table, in order to reach target spaces that are located on the side of the operating table.

### Safe position of the robotic arm

A potential issue with a neck extending laterally outwardly from the movable cart is that in certain embodiments, the robotic arm, being outside the footprint of the movable cart, may enter in collision with an obstacle during transport, thus potentially threatening its mechanical integrity. By footprint is meant in the present text a vertical projection of the cross section of the movable cart onto a horizontal plane.

According to an embodiment, the mechanical coupling between the neck and the movable cart is configured to enable movements of the neck of the robotic system between an operative position wherein the shoulder of the robotic arm is located at the desired position along the Ax axis in view of implementing the surgical intervention and a retracted position wherein the robotic arm is within the footprint of the movable cart in view of transportation and/or storage of the surgical robotic system. In a preferred embodiment, the retracted position is obtained by rotating the base of the neck by 180 degrees.

This condition is already met by embodiments previously described, such as a mechanical coupling between the neck and the movable cart enabling a rotation of the neck relative to the movable cart around a vertical axis, such as depicted in FIG. 4.

Preferably, in transportation mode, the neck is within the footprint of the movable cart as well. In the situation of FIG. 4, the frontal side defines a vertical plane PP which partially delimits the footprint, the neck and robotic arm being located on the inner side of the footprint.

### Safety fence

As other embodiments of the invention may place the robotic arm outside the footprint of the movable cart even when the surgical robotic system is not used for surgery, notably during transportation of the surgical robotic system, said robotic arm may enter in collision with an obstacle, thus potentially threatening its mechanical integrity.

Therefore, according to an embodiment of the invention, the surgical robotic system further comprises a safety fence movable between an extended protective position wherein the safety fence protrudes outwardly relative to the robotic arm and a retracted position wherein the safety fence enables operation of the robotic arm.

Therefore, any obstacle met during transportation by the robotic system would enter in collision with the safety fence rather than the robotic arm.

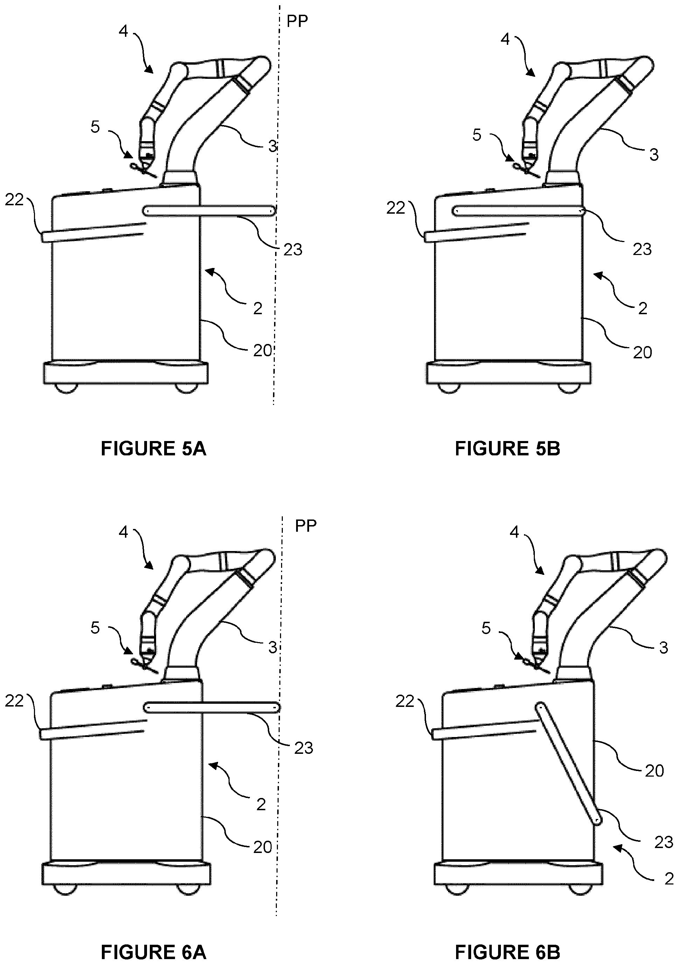

FIGS. 5A and 5B illustrate a first embodiment of such a safety fence 23, respectively in an extended configuration suitable for transportation of the surgical robotic system and in a retracted configuration suitable for operation of the surgical robotic system. The safety fence 23 is slidable relative to lateral sides of the movable cart in a substantially horizontal plane. As shown in FIG. 5A, in the extended configuration, the safety fence defines a vertical plane PP which extends outwardly relative to the frontal side 20 and partially delimits the footprint of the movable cart. Since the robotic arm and the neck are located on the inner side of the footprint, they may not be hurt by an obstacle hit by the safety fence 23. In the retracted configuration, the safety fence is arranged in such a way as to enable placing the frontal side 20 along a side of the operative table (which is not illustrated in these figures).

FIGS. 6A and 6B illustrate a second embodiment of such a safety fence 23, respectively in an extended configuration suitable for transportation of the surgical robotic system and in a retracted configuration suitable for operation of the surgical robotic system. The safety fence 23 is pivotable relative to lateral sides of the movable cart toward the bottom of the movable cart about a horizontal axis. As shown in FIG. 6A, in the extended configuration, the safety fence defines a vertical plane PP which extends outwardly relative to the frontal side 20 and partially delimits the footprint of the movable cart. Since the robotic arm and the neck are located on the inner side of the footprint, they may not be hurt by an obstacle hit by the safety fence 23. In the retracted configuration, the safety fence is arranged in such a way as to enable placing the frontal side 20 along a side of the operative table (which is not illustrated in these figures).

Of course, other designs of safety fences may be used to protect the robotic arm.

### Safety parameters

According to an embodiment of the invention, the control unit is further configured to detect at least one safety parameter relative to the surgical robotic system.

Said safety parameter indicates the ability to preserve the integrity of the surgical robotic system, and more particularly of the robotic arm, during transportation and/or storage of the surgical robotic system.

According to an embodiment, at least one safety parameter comprises the extended position of safety fence.

According to another embodiment, at least one safety parameter comprises the position of the neck and robotic arm within the footprint of the movable cart.

According to a preferred embodiment, the displacement of the surgical robotic system may be possible only when the control unit detects that said at least one safety parameter is activated.

In a preferred embodiment, if the user forgets to activate the safety mechanism for transportation of the robotic system to another location inside the hospital, an alarm is activated when the system has detected that a certain distance has been made by the mobile cart, for example two meters. This can be achieved by placing sensors on the wheels of the mobile cart. It is also possible to block the motion of the mobile cart if the alarm has been activated and the user has not corrected the default condition.

## Claims

1. Surgical robotic system (1) to reach a plurality of surgical targets inside a target space, said system comprising:
- a robotic arm (4) having at least six degrees of freedom, the robotic arm comprising a base (40) located at a proximal end and a plurality of consecutive segments interconnected by a plurality of joints extending from the base (40) to a distal end, and said robotic arm having a first joint center (O) between the base (40) and the first segment (41),
- an end effector (5) for holding a medical device such as a surgical instrument or a surgical tool guide, said end effector being mechanically coupled to the robotic arm distal end,
- a movable cart (2) having a frontal side (20) comprising at least one frontal surface configured to be placed against a side (60) of an operating table (6), defining a horizontal axis (Ax) with a direction (x) orthogonal to the frontal surface and an origin point A located on the frontal surface such that all points located on the movable cart side have a negative coordinate on said axis (Ax), and all points located on the operating table side have a positive coordinate on said axis (Ax),
- a rigid elongated neck (3) comprising a proximal end mechanically coupled to the movable cart and a distal end mechanically coupled to the first segment (41) of the robotic arm,
- a control unit (7) configured to controllably move the robotic arm,
**characterized in that** the neck (3) is configured such that the coordinate (K) of the first joint center (O) on the axis (Ax) is strictly positive.

2. The surgical robotic system according to claim 1, wherein the neck (3) is configured such that the coordinate (K) of the first joint center (O) on the axis (Ax) is greater than +100 mm.

3. The surgical robotic system according to claim 1 or claim 2, wherein the neck (3) is configured such that a coordinate of a shoulder point (S), which is defined as the intersection point between the first and second axes of the robotic arm, along the axis (Ax) is comprised between minimum and maximum coordinates of the target space on the axis (Ax).

4. The surgical robotic system according to any of claims 1 to 3, wherein the mechanical coupling between the neck (3) and the movable cart (2) is configured to enable a movement of the neck relative to the movable cart according to at least one degree of freedom.

5. The surgical robotic system according to claim 4, wherein the mechanical coupling between the neck (3) and the movable cart (2) is configured to enable a translation of the neck along a vertical axis (z) and a rotation of the neck (3) about said vertical axis (z) relative to the movable cart (2).

6. The surgical robotic system according to any one of claims 4 or 5, wherein the mechanical coupling between the neck (3) and the movable cart (2) comprises at least one motor configured to drive a movement of the neck relative to the movable cart according to said at least one degree of freedom.

7. The surgical robotic system according to any one of claims 4 to 6, wherein the mechanical coupling between the neck (3) and the movable cart (2) is configured to enable a motorized vertical translation of the neck (3) and a manual rotation of the neck (3) around the vertical axis (z).

8. The surgical robotic system according to claim 6 or claim 7, wherein the control unit is also configured to controllably move the motorized motions of the cart-neck mechanical coupling.

9. The surgical robotic system according to any of claims 4 to 8, wherein the mechanical coupling (30) between the neck and the movable cart comprises at least one abutment and at least one locking means (31) cooperating with the abutment to stop and lock the neck relative to the movable cart in a determined configuration.

10. The surgical robotic system according to any one of claims 1 to 9, wherein the control unit is further configured to detect at least one safety configuration of the movable cart and/or robotic system.

11. The surgical robot according to any one of claims 4 to 9 in combination with claim 10, wherein the at least one safety configuration comprises the position of the neck (3) and robotic arm (4) within a footprint of the movable cart.

12. The surgical robot system according to any one of claims 10 or 11, further comprising a safety fence (23) movable between an unfolded protective position wherein the safety fence surrounds the robotic arm and a folded position wherein the safety fence enables operation of the robotic arm, wherein the at least one safety parameter comprises the unfolded position of safety fence.

13. The surgical robotic system according to any one of claims 10 to 12, wherein displacement of the movable cart (2) on a distance greater than two meters is possible only when the control unit detects that said at least one safety configuration is activated.

14. The surgical robotic system according to any one of claims 1 to 13, wherein the neck (3) comprises a curved portion between its proximal end and its distal end.

15. The surgical robotic system according to any one of claims 1 to 14, wherein the neck distal end has an axis (N) aligned with the first axis (A1) of the robotic arm (4).
